# EUROPEAN PATENT SPECIFICATION

(11) **EP 4 120 224 B1**
(45) Date of publication and mention of the grant of the patent: **06.08.2025**
(21) Application number: 21768107.1
(22) Date of filing: 09.03.2021
(51) Int. Cl.: G09B 23/28, G09B 23/30, G09B 23/34, A61N 1/375, A61N 1/372

(54) **CATHETER SIMULATOR AND HEART MODEL FOR CATHETER SIMULATOR**
KATHETERSIMULATOR UND HERZMODELL FÜR EINEN KATHETERSIMULATOR
SIMULATEUR DE CATHÉTER ET MODÈLE CARDIAQUE POUR SIMULATEUR DE CATHÉTER

(30) Priority: 11.03.2020 JP 2020042024
(43) Date of publication of application: 18.01.2023
(73) Proprietor: The University of Osaka, Suita-shi Osaka 565-0871 (JP); JMC Corporation, Yokohama-shi, Kanagawa 222-0033 (JP)
(72) Inventor: OKAYAMA, Keita, Suita-shi, Osaka 565-0871 (JP); SAKATA, Yasushi, Suita-shi, Osaka 565-0871 (JP); WATANABE, Daichi, Yokohama-shi, Kanagawa 222-0033 (JP); INADA, Makoto, Yokohama-shi, Kanagawa 222-0033 (JP)
(74) Representative: advotec.
(86) International application number: PCT/JP2021/009161
(87) International publication number: WO 2021/182436

(56) References cited:
- EP-A1- 3 279 885
- WO-A1-2018/079711
- JP-A- 2014 170 075
- JP-A- 2014 501 584
- JP-A- 2016 057 451
- JP-A- 2017 040 906
- JP-A- 2018 153 626
- JP-A- 2019 150 571
- US-A1- 2019 147 768

## Description

### TECHNICAL FIELD

The present invention relates to a catheter simulator and a heart model used for this catheter simulator.

### BACKGROUND ART

Conventionally, it has been known to perform an operation to install a pacemaker in the body for a patient having a heart disease such as arrhythmia. A conventional pacemaker includes a main body having a battery and an electric circuit built-in; and a lead (lead wire) led out from the main body. When installing such a pacemaker in a human body, an implantation method is employed in which the main body is subcutaneously implanted and a lead extending from the main body is inserted into a vein and caused to reach the heart. By installing a pacemaker and applying electrical simulation to the heart, it is possible to correct rhythmic abnormalities in arrhythmia.

With regard to a pacemaker such as described above, since a subcutaneous pocket is formed in the skin and the main body is installed therein, there is a possibility that the main body or a lead placed in a vein may cause infection or various complications. Therefore, recently, small pacemakers (leadless pacemakers) that can be placed in the heart by reducing the size of the main body, have been used. For example, Medtronic plc is manufacturing and selling a small leadless pacemaker (MICRA; registered trademark) in which an electrical circuit, electrodes, and a battery are incorporated into a cylindrical main body having a volume of about 1 cc. This leadless pacemaker is installed, not by being implanted in the form of making an incision in the patient's skin as in conventional cases, but by directly conveying the main body into the heart (right ventricle) by means of a catheter.

The above-described catheter includes a holding part (device cup) that holds a leadless pacemaker at the tip, and maneuvers for inserting the catheter mainly through the inferior vena cava in the groin area, conveying the device cup to the right ventricle via the right atrium, and locking the main body of the leadless pacemaker to the interventricular septum between the right ventricle and the left ventricle, have been adopted. Specifically, four tines (locking part) each formed into a hook shape and having elasticity are provided on the tip side of the main body, and these tines are caused to sink into the myocardium by pressing the tines against the myocardium (interventricular septum) with a constant pressing force and releasing the pressing force by manipulating the handle at hand of the catheter, so that the main body is fixed.

With regard to the operation for implanting a leadless pacemaker using a catheter such as described above, complications such as a hole in the wall of the heart, which is called perforation, are known to occur, and death cases have also been reported. For that reason, it is considered desirable that maneuvers can be trained by using a simulator in order to promote acquisition or proficiency of manipulating techniques; however, at the moment, there is no proposed simulator for training of the catheter maneuvers for a leadless pacemaker. The inventors of the invention have proposed, in a previous patent application (Patent Document 1), a simulator including a heart model according to the types of heart diseases; a container holding the heart model; and a pump circulating a stream of water to the container, so that improvement of catheter maneuvers for various heart diseases can be promoted. With this simulator, maneuvers similar to the actual operations for various heart diseases can be practiced by storing water in a small container to float a heart model, creating a flow into the heart with a pump to generate a pulsatile flow, inserting a catheter into the floating heart model, and manipulating the catheter.

### CITATION LIST

### PATENT DOCUMENT

Patent Document 1: PCT/JP2016/057000, also published as EP 3 279 885 A1

### SUMMARY OF THE INVENTION

### PROBLEM TO BE SOLVED BY THE INVENTION

The simulator disclosed in Patent Document 1 is effective for enhancing the skill of operations performed for actual heart diseases; however, maneuvers for installing a leadless pacemaker such as described above in the heart cannot be practiced. That is, conventional simulators are not intended for an operation of installing a leadless pacemaker in the heart, and even for such an operation, it is desirable to provide a heart model and a simulator that can promote enhancement of maneuvers.

The invention was achieved by focusing attention on the above-described actual circumstances, and it is an object to provide a heart model that enables the training of catheter manipulation for installing a leadless pacemaker in the heart to be conveniently performed, and a catheter simulator for installing such a heart model.

### MEANS FOR SOLVING PROBLEM

In order to achieve the above-described object, a heart model according to the invention has a main body having one or more cavities in the inner part; a simulated blood vessel passage being contiguous to the main body and allowing insertion therethrough of a catheter; and a holder being detachably installed inside the main body and including a flexible part.
The flexible part is formed from a porous material having surface unevenness on a surface thereof or from a soft resin mounted on the holder, wherein the holder including the flexible part is detachably installed at a position of an interventricular septum inside the main body. The one or more cavities include a right atrium, and a right ventricle and a left ventricle divided by the interventricular septum. The simulated blood vessel passage comprises a superior vena cava or an inferior vena cava, or both of them, communicating with the right atrium. In the interventricular septum, an opening detachably fitting the holder is formed such that the flexible part is exposed inside the right ventricle, and in an outer shell of the main body, an opening part for holder attachment and detachment is formed to allow the holder to be installed in the opening of the interventricular septum.

Furthermore, the heart model according to the invention is used when performing a simulation of an operation for installing a leadless pacemaker. The heart model has a main body formed from an elastic material and having a right atrium and a right ventricle in the inner part; a simulated blood vessel passage provided in the main body and allowing insertion therethrough of a catheter holding a leadless pacemaker; and a holder being detachably installed inside the main body and including a flexible part capable of locking a locking part of the leadless pacemaker.

Furthermore, the heart model according to the invention is used when simulating myocardial biopsy maneuvers. The heart model has a main body formed from an elastic material and having a right atrium and a right ventricle in the inner part; a simulated blood vessel passage provided in the main body and allowing insertion therethrough of biopsy forceps and a sheath serving as a passage for the biopsy forceps; and a holder detachably installed inside the main body and including a flexible part capable of locking tip parts of the sheath and the biopsy forceps.

The heart model having the above-described configuration can be installed in a dry environment or can be installed in a state of floating in an environment filled with a liquid such as water, so that various operations related to the heart can be practiced. It is an object of the invention to provide a simulator capable of conducting such a catheterization operation, and the simulator has a container equipped with an accommodation part capable of accommodating a heart model of the above-described configuration in a state of being filled with a liquid; a connection part provided in the container and holding the heart model; and an introduction part provided in the container and allowing insertion therethrough of a catheter, in which the container includes a holding part for holding the heart model such that the simulated blood vessel passage is connected to the introduction part, and catheter manipulation can be practiced in the heart model held by the connection part.

### EFFECT OF THE INVENTION

According to the heart model and the catheter simulator of the invention, it is possible to practice an operation of installing a leadless pacemaker in the heart with a simple configuration.

### BRIEF DESCRIPTION OF DRAWINGS

Fig. 1 is a diagram illustrating an embodiment of a catheter simulator according to the invention;
Fig. 2 is a diagram illustrating the state in which a heart model according to the invention is installed in a container of the catheter simulator shown in Fig. 1;
Fig. 3 is a diagram illustrating the container of the catheter simulator shown in Fig. 1;
Fig. 4 is a diagram illustrating an example of the heart model according to the invention;
Fig. 5 is a diagram schematically illustrating the outline configuration of the inner part of the heart model shown in Fig. 4;
Fig. 6 is diagrams illustrating a holder that is detachably attached inside the heart model, while Fig. 6(a) is a diagram illustrating the holder alone, and Fig. 6(b) is a diagram illustrating the holder and a flexible part attached to the holder;
Fig. 7 is a diagram illustrating the heart model in the state before the holder shown in Fig. 6 is attached;
Fig. 8 is a diagram illustrating the heart model to which the holder shown in Fig. 6 is attached;
Fig. 9(a) is a diagram illustrating a first Modification Example of the flexible part, Fig. 9(b) is a schematic diagram illustrating the situation in which a pacemaker is inserted, and Fig. 9(c) is a diagram illustrating a second Modification Example of the flexible part;
Fig. 10(a) is a diagram illustrating a third Modification Example of the flexible part, and Fig. 10(b) is a schematic diagram illustrating the situation in which a pacemaker is inserted;
Fig. 11 is diagrams illustrating an example of a leadless pacemaker, while Fig. 11(a) is a perspective view, and Fig. 11(b) is a diagram illustrating the state at the time of installation; and
Fig. 12 is a schematic diagram illustrating the access route of a catheter toward the flexible part of the holder installed inside the heart model, and the state in which a leadless pacemaker is locked.

### MODE(S) FOR CARRYING OUT THE INVENTION

Hereinafter, embodiments of the invention will be described with reference to the drawings.

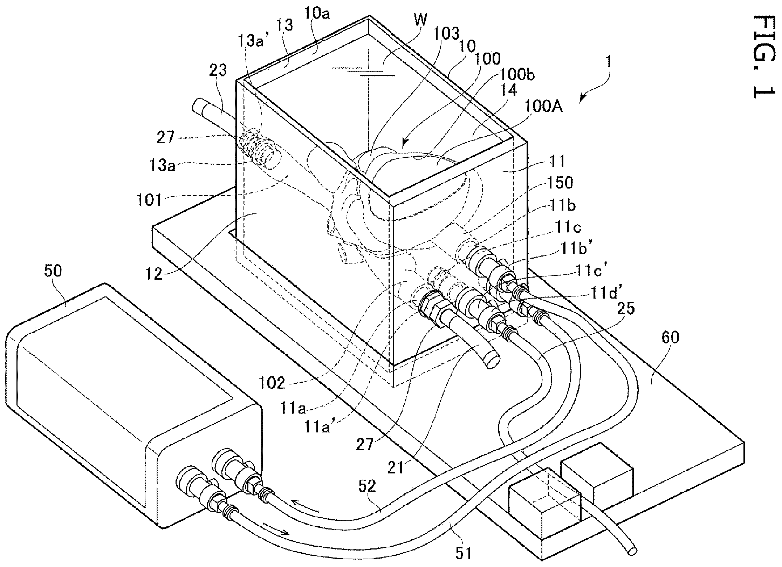

Fig. 1 is a diagram illustrating an embodiment of a catheter simulator according to the invention, and a heart model used therein. A catheter simulator 1 includes a container 10 accommodating a heart model 100; and a pump 50 (pulsatile flow generating pump) for circulating a liquid in a state in which the container 10 is filled with a liquid W such as water. The container 10 is installed so as not to be movable on a base 60, in order to promote stabilization of installation.

The catheter simulator 1 according to the present embodiment includes a pump 50 so that a liquid is circulated inside the installed heart model, and a pulsatile flow by means of blood, which is equivalent to an actual heart, can be generated. The pump 50 is configured to be intermittently driven so that a pulsatile flow is generated for the liquid filled in the container 10. Specifically, when a pulsation in the human body that can be assumed is considered, a heart rate of 20 to 200 bpm (beat per minute) is sufficient. With regard to actual cardiac operations, since it is speculated that most cardiac operations are performed at a heart rate in the range of about 40 to 100 bpm, the capacity of the pump 50 may have a specification that can send 20 to 200 times per minute of pulsatile flow to the heart model, and it is possible to perform an effective simulation with a pump that can send at least 40 to 150 times per minute of pulsatile flow to the heart model.

Incidentally, on the occasion of simulating catheter manipulation, the pump 50 is not necessarily a required element. For example, it is possible to practice catheter manipulation even in a state of having the container 10 filled with a liquid or in a state of holding a heart model in the container.

As described above, the inventors of the invention have proposed a four-cavity model, a coronary artery model, and a TAVI (Transcatheter Aortic Valve Implantation) model as heart models in a previous patent application, PCT/JP2016/057000. These heart models are formed from materials having elasticity close to that of an actual human heart, for example, one alone or a combination of a plurality of PVA (polyvinyl alcohol), polyurethane, an epoxy resin, an unsaturated polyester, a phenol resin, a silicone, materials similar to these; and other thermosetting resins and thermoplastic resins, so that catheter manipulation is practiced with a tactile sensation close to that of human organs.

The heart model 100 according to the invention is configured in a dedicated form in which maneuvers related to installation of a leadless pacemaker, maneuvers for collecting myocardial tissue by using a catheter, such as myocardial biopsy, and the like, all of which cannot be practiced with the above-described known heart models, can be practiced. Preferably, the heart model is formed by means of a material similar to that of the above-described heart model, so that during a simulation, a feeling of touch at the time of catheter manipulation close to reality can be obtained. In this case, with regard to the color of the heart model 100, the inner part may be made visually not recognizable by adopting the same color as that of an actual heart, so that the trainee can carry out simulation while irradiating X-radiation and observing a monitor. Alternatively, a transparent or translucent color may be adopted so that the trainee can carry out simulation while directly observing, by visual inspection, the movement of a catheter, a guide wire, and other devices, which are inserted. Incidentally, even when the trainee forms a heart model with a material that is visually recognizable, by covering the container 10 with a cover or the like so that the heart model cannot be seen, it is also possible to figure out the behavior of a catheter only on the monitor by viewing by means of X-ray fluoroscopy.

It is preferable that the heart model 100 of the invention is formed integrally without having any artificial seams. As a result, generation of a flow of a liquid (blood flow) that is not seen in the human body due to seams can be prevented. Furthermore, in addition to the effect that blocking of the field of vision by seams at the time of inserting a catheter can be prevented, the appearance of unnatural shadows under X-ray fluoroscopy can be avoided.

As a method of forming a heart model by using a material that satisfies properties such as mentioned above, for example, it is possible to use an optical shaping method (JP 5236103 B2) invented by the present Applicant. When the shaping method is used, a highly accurate heart model for each patient can be formed at relatively low cost in a short period of time, based on the imaging data (cardiac CT data) of a human organ. Therefore, the trainee can create a patient-specific heart model and receive simulation training for catheter manipulation, in advance of an actual operation. Furthermore, it is also possible to utilize the catheter simulator according to the invention as a preliminary preparation prior to actual catheter manipulation, such as selecting and examining a catheter or various devices optimal for the patient before an examination or an operation.

When the heart model 100 is formed according to the above-described optical shaping method, since a state close to the human body can be reproduced, the surface of the heart model is not smooth and includes slight surface unevenness, just like the human body. In this case, even when the heart model is formed by means of a transparent or translucent material such as described above, since visible light is diffusely reflected on the surface having surface unevenness, visibility may be deteriorated. In this case, after the heart model is formed, diffuse reflection can be reduced by coating the surface with the same material to smoothen the surface having surface unevenness, and visibility can be improved.

Furthermore, the heart model 100 of the present embodiment and the catheter simulator 1 using that heart model are configured such that a liquid is circulated in the container 10 by the pump 50, and a pulsatile flow is caused to flow inside the heart model. As a result, the main body having elasticity repeatedly undergoes expansion and contraction, and therefore, the liquid can flow as is the case of the blood flow of an actual heart. By circulating a liquid as such, in a simulation utilizing a contrast agent, the contrast agent can be suppressed from staying inside the heart, and the behavior of the catheter or the leadless pacemaker can be figured out on the monitor.

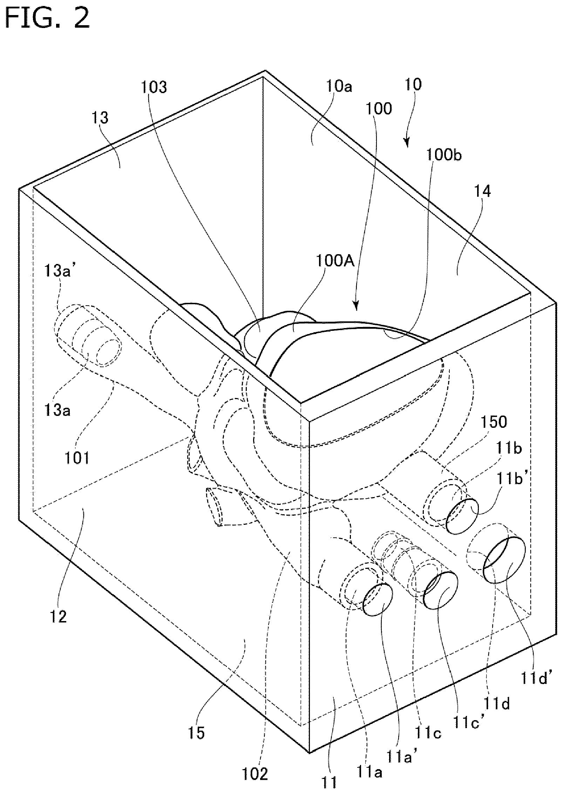

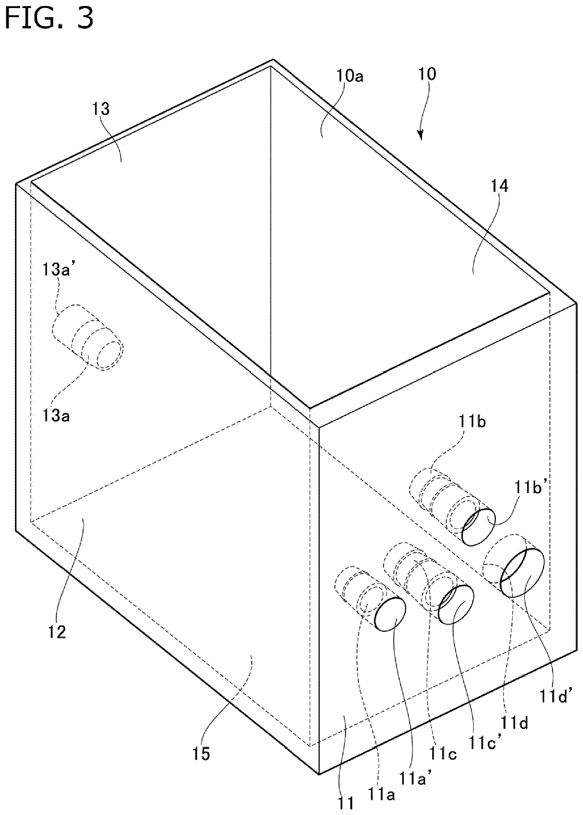

Next, with reference to Fig. 2 and Fig. 3 in addition to Fig. 1, the configuration of the container 10 of the catheter simulator where the heart model 100 is installed, and the heart model 100 will be described.

Incidentally, regarding the container 10 and the heart model 100 that will be described in the following embodiment, a four-cavity type including a right atrium, a right ventricle, a left atrium, and a left ventricle in the inner part of the main body of the heart model will be described as an example, so that the maneuvers related to installation of a leadless pacemaker can be effectively practiced. However, it is not necessarily required that four cavities are provided in the heart model, and it is acceptable to have one or more cavities provided.

The container 10 of the present embodiment includes an accommodation part 10a that accommodates a liquid W such as water and an aqueous electrolyte by means of side walls 11 to 14 of four faces and a bottom face 15. On the side wall 11, connection parts 11a and 11b that can be held by connecting one end side of the heart model 100 in a state in which the accommodation part 10a is filled with a liquid, are provided so as to protrude into the accommodation part. Furthermore, on the side wall 13, a connection part 13a that can be held by connecting the other end side of the heart model 100 is provided so as to protrude into the accommodation part. That is, the connection parts 11a, 11b, and 13a are configured to be cylindrical in shape and protrude into the accommodation part, and by thrusting a tubular-shaped part (constituting a simulated blood vessel passage or the like) of the heart model 100 into that portion, the heart model 100 is held in a state of floating in the container filled with a liquid. Incidentally, the connection parts 11a and 13a also have a function as introduction parts through which a catheter is introduced from the outside. That is, the connection parts 11a and 13a are integrated with introduction parts 11a' and 13a' coaxially protruding to the outside of the container, and introduction tubes 21 and 23 of catheters are connected to the introduction parts, respectively. In this case, the introduction tubes 21 and 23 each have a catheter introduction terminal at the tip part on the external side of the container 10. Each of the introduction terminals has a function (valve function) of preventing the liquid filled in the introduction tubes 21 and 23 from leaking to the outside and also has a structure in which the trainee can introduce a catheter into the introduction tubes 21 and 23 and pull out the catheter therefrom.

In the present embodiment, a superior vena cava 101 and an inferior vena cava 102 communicating with the right atrium are formed as a simulated blood vessel passage in the main body 100A of the heart model 100 so as to protrude from the main body 100A, as in an actual heart. Then, by fitting these superior vena cava 101 and inferior vena cava 102 into the connection parts 11a and 13a, respectively, the heart model 100 can be held so as to float in the accommodation part. In this case, as a heart model, an aorta (simulated blood vessel passage) 103 communicating with the left ventricle may be formed in the main body 100A, as in an actual heart. By further providing a connection part 11c on the side wall 11 of the container 10 so as to protrude to the inside and fitting the aorta 103 into this connection part to be connected, the heart model 100 can be held more stably. Furthermore, an introduction part 11c' may be formed in the connection part 11c as in the above-described connection parts 11a and 13a, and an introduction tube 25 of a catheter may be connected to this portion.

The connection part 11b is intended for circulating the liquid in the accommodation part and generating a pulsatile flow inside the heart model 100 held therein, and an inflow port 150 formed to protrude at the apex of the main body 100A of the heart model 100 is connected to the connection part 11b. This inflow port 150 is an element that does not exist in an actual heart, and has a function of causing a pulsatile flow to flow into the internal space (space on the left ventricle) of the main body 100A, as an inflow tube 51 supplying a liquid from the pump 50 is connected to an inflow tube 11b' that protrudes to the outside coaxially with the connection part 11b.

Furthermore, on the side wall 11, an outlet port 11d for discharging the liquid in the accommodation part 10a to the pump 50 side is formed. This outlet port 11d is provided with an outlet tube 11d' coaxially protruding to the outside. As an outflow tube 52 that causes the liquid to the pump 50 side is connected to this outlet tube 11d', the liquid in the accommodation part 10a is circulated via the pump 50.

The side walls 11 to 14 and the bottom face 15 may be formed by means of a material having a strength that can stably accommodate the liquid and the heart model, and the container 10 may be formed into a shape that can stably accommodate the liquid and the heart model. Furthermore, it is preferable that the material for the side walls 11 to 14 and the bottom face 15 constituting the container has transparency. As the side walls and the bottom face have transparency, it is possible to observe the behavior of the heart model installed in the container 10, the catheter to be inserted from the outside of the container 10, and the like by visual inspection during a simulation. Examples of a material that has such strength and is transparent include acrylic, polycarbonate, PET, and polystyrene.

Incidentally, even when the container 10 is formed from a material that can be visually recognized by the trainee, a camera may be installed to display the container 10 on a monitor or the like, or X-ray fluoroscopy may be performed to display the container 10 on a monitor or the like. According to such a configuration, a simulation of figuring out the behavior of a catheter only on a monitor can be carried out, and it is also possible to realize a state closer to reality. Furthermore, the use of visual recognition, confirmation of monitor display, and X-ray imaging can be selected according to the stage and content of the training.

The upper part of the container 10 is opened, and a lid that can be opened and closed may be disposed here. As a result, when performing preparation for training and cleaning up, such as an operation of filling the accommodation part 10a with a liquid and an operation of installation a heart model in the liquid, the operations can be efficiently carried out through the opening at the top face of the container. Furthermore, by making the lid transparent, intrusion of dust can be prevented. In addition, a decrease in visibility caused by shaking of the liquid surface can be prevented by bringing the lid into close contact with the liquid surface.

The connection parts 11a and 13a have the functions as introduction parts for a catheter in addition to the function of supporting the heart model, and a communication hole through which a catheter is inserted is formed at each of the connection parts. As described above, introduction tubes 21 and 23 through which a catheter manipulated by the trainee is introduced from the outside of the container 10, are connected to the introduction parts 11a' and 13a', respectively, which protrude coaxially with the connection parts 11a and 13a to the outside. These introduction tubes 21 and 23 include a connection mechanism that can be manipulated from the outside of the container 10. The connection mechanism has a structure in which, for example, when introduction tubes 21 and 23 are thrusted into the introduction parts 11a' and 13a' and a manipulation member (a nut or the like) 27 is rotated, the introduction tubes 21 and 23 can be fixed or released, so that detaching and attaching manipulation of the introduction tubes can be easily carried out.

Incidentally, a configuration in which when the aorta is fitted into the connection part 11c to be connected, the connection part 11c has a function as an introduction part for a catheter as in the case of the connection parts 11a and 13a, may also be adopted. Furthermore, as described above, the heart model 100 of the present embodiment has a configuration in which a liquid is supplied from the pump 50 through the inflow port 150, the liquid inside the accommodation part is returned to the pump 50 side through the outlet tube 11d' and the outflow tube 52, and a pulsatile flow flows in the heart model 100.

It is preferable that the connection part 11b (inflow tube 11b') and the outlet port 11d (outlet tube 11d') are provided with a valve for opening and closing (not shown in the diagram). This valve for closing has a function of preventing the liquid in the accommodation part 10a from leaking out of the container 10, by being closed when the pump 50 is detached from the container 10 after completion of the simulation.

With regard to actual simulation, the accommodation part 10a is filled with a liquid W, and the heart model 100 is installed in a state of floating in the liquid. As the heart model 100 is in a floating state, the trainee can obtain a feeling of touch closer to reality at the time of catheter manipulation. Incidentally, it is also acceptable that without providing the side walls with the connection part protruding to the inside, for example, a dedicated holder is installed at the bottom face of the container so as to support the heart model 100 from below and hold the heart model 100 in the liquid.

Since the elements accommodated in the container 10 are limited only to a heart model having a size similar to that of the human heart and a liquid for allowing the heart model to float therein, the container 10 can be miniaturized. The external dimension of the container 10 according to the present embodiment is about 20 cm × 20 cm × 15 cm, and the amount of the liquid (water) required for filling the container is approximately limited to about 3 L to 6 L. When the container 10 is miniaturized, waste of space in the place where the simulation is performed can be eliminated, and the storability and transportability of the container 10 and the catheter simulator using the container 10 can be enhanced. Furthermore, since the amount of water to be filled in the accommodation part 10a of the container is limited to about 6 L, even at a place where water supply cannot be utilized, simulation can be performed by transporting water with a tank or the like, and the range of selection for the place of implementation is widened. In addition, since the weight of the container filled with water is light to the extent that the trainee can handle alone, preparation of the simulation and cleaning up can be carried out without the constraints of assistants.

Incidentally, the above-described catheter simulator 1 and the container 10 have structures that are suitably mainly for practicing the maneuvers related to installation of a leadless pacemaker as follows; however, regarding the container, a container in which the various heart models disclosed in the previous patent applications can be installed, can also be utilized.

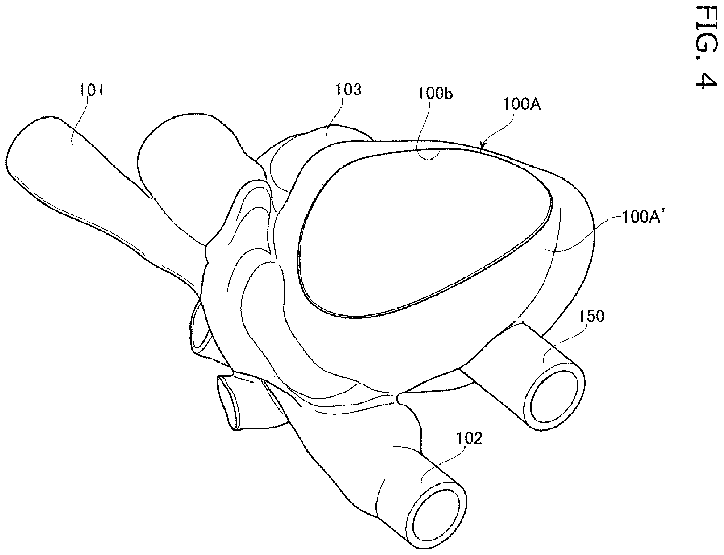

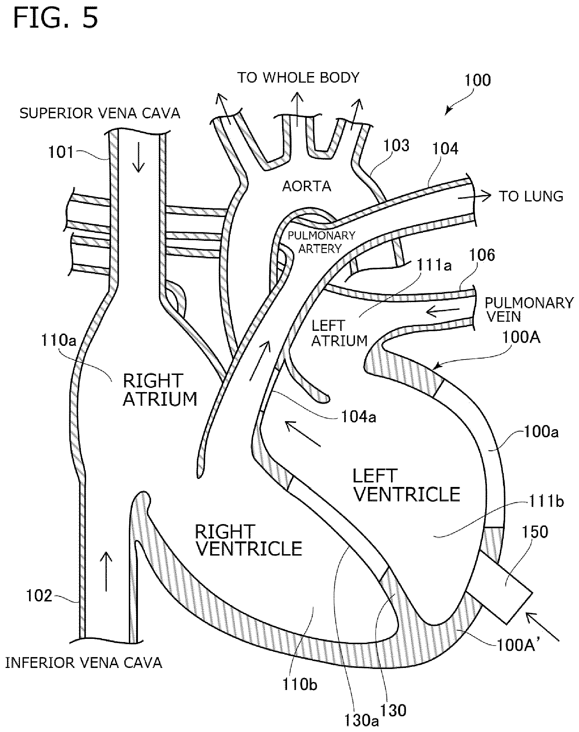

Next, the configuration of the heart model 100 according to the present embodiment will be described with reference to Fig. 4 and Fig. 5.

The heart model 100 of the present embodiment is formed in consideration of approaching a leadless pacemaker from the inferior vena cava by means of a catheter as in the operations generally performed, and installing the leadless pacemaker on the right ventricle side of the interventricular septum between the left ventricle and the right ventricle.

The heart model 100 includes a main body 100A having the same shape as the human heart, and in the inner part of the main body 100A, a right atrium 110a, a right ventricle 110b, a left atrium 111a, and a left ventricle 111b are formed as in an actual heart. Vena cava (superior vena cava 101 and inferior vena cava 102) extend from the right atrium 110a, and a pulmonary artery 104 extends from the right ventricle 110b. The inferior vena cava 102 is connected to the connection part 11a (introduction part 11a') formed in the container 10 and serves as an introduction port for a catheter. Furthermore, the superior vena cava 101 is connected to the connection part 13a formed in the container 10.

In the actual human body, the inferior vena cava 102 reaches the femoral vein running in the groin part and serves as an introduction route for a catheter that is introduced through the groin part (base of the legs). Furthermore, the superior vena cava 101 reaches the internal jugular vein running along the base of the neck. In this case, the superior vena cava 101 and the connection part 13a connected thereto may also be used as introduction routes for the catheter. That is, since an approach through the superior vena cava 101 can also be considered according to the configuration of the leadless pacemaker, the superior vena cava 101 may be used as a simulated blood vessel passage through which a catheter is inserted.

As mentioned above, in the present embodiment, an inflow port 150 that does not exist in an actual heart is formed at the apex (lower end part on the left ventricle side) of the outer shell 100A' of the main body 100A, and as the inflow port 150 is connected to the connection part 11b of the container 10, the heart model 100 is held in a state of stably floating, while a pulsatile flow is made to flow in the inside. Incidentally, in a configuration in which a pulsatile flow flows as such, a liquid communication part 104a opened to the left ventricle 111b may be formed in the pulmonary artery 104 communicating with the right ventricle 110b.

In the main body 100A, an aorta 103 extends from the left ventricle 111b as in an actual heart. This aorta 103 may be lengthened and connected to the connection part 11c of the container 10 or may have a configuration of being opened in the accommodation part of the container. Alternatively, a liquid communication part (not shown in the diagram) may be formed in the pulmonary artery 104 such that a liquid communicates between the pulmonary artery 104 and the aorta 103. Furthermore, in the main body 100A, a pulmonary vein 106 extends from the left atrium 111a as in an actual heart. The pulmonary artery 104 and the pulmonary vein 106 are opened in the accommodation part of the container. Incidentally, in an actual heart, there exist a tricuspid valve between the right atrium 110a and the right ventricle 110b, a pulmonary valve between the right ventricle 110b and the pulmonary artery 104, a mitral valve between the left atrium 111a and the left ventricle 111b, and an aortic valve between the left ventricle 111b and the aorta 103; however, the main body 100A of the present embodiment is formed by omitting these valves. Furthermore, when the main body is formed by omitting these valves, the arrangement structure of cavities can be appropriately transformed, such as adopting a structure in which an integrated cavity is made to look like the left atrium/left ventricle and the right atrium/right ventricle.

As described above, a leadless pacemaker is conveyed by a catheter via the inferior vena cava 102, the right atrium 110a, and the right ventricle 110b. At the interventricular septum 130 that is the actual position of installation, a detachable holder capable of locking a locking part (tines) of the leadless pacemaker that will be described below, is installed.

Hereinafter, the configuration, action, and installation mode of a detachable holder will be described with reference to Fig. 5 to Fig. 8.

As described above, a heart model is formed from one alone or a combination of a plurality of PVA, polyurethane, an epoxy resin, an unsaturated polyester, a phenol resin, silicon, materials similar to these; and other thermosetting resins and thermoplastic resins. Accordingly, from the viewpoint of passability of the catheter, the surface can be smoothly finished, and even when a leadless pacemaker is conveyed to a predetermined location by means of a catheter, it is not possible to fix the locking part (thrusting the locking part into the interventricular septum to be fixed).

In contrast, an actual heart is such that the surface portion has flexibility and has a concavo-convex shape, and the leadless pacemaker can be fixed (placed) by thrusting the locking part without slipping from the conveyed portion.

In the invention, among the constituent elements of the heart model 100 formed from the above-described materials, only the portion where the leadless pacemaker is actually locked (when the catheter maneuver is carried out, the portion that is the object of manipulation) can be exchanged, and at the same time, the portion is formed from a material capable of locking the locking part of the leadless pacemaker. That is, it is configured that a holder 200 which is formed from a material different from the material forming the heart model and includes a surface structure (here, referred to as flexible part) to which the locking part of the leadless pacemaker can be locked, can be detachably installed at a predetermined portion.

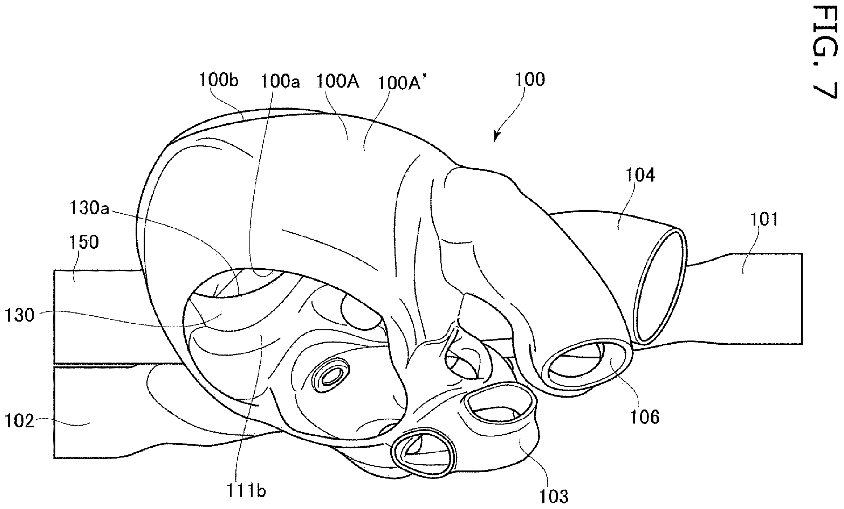

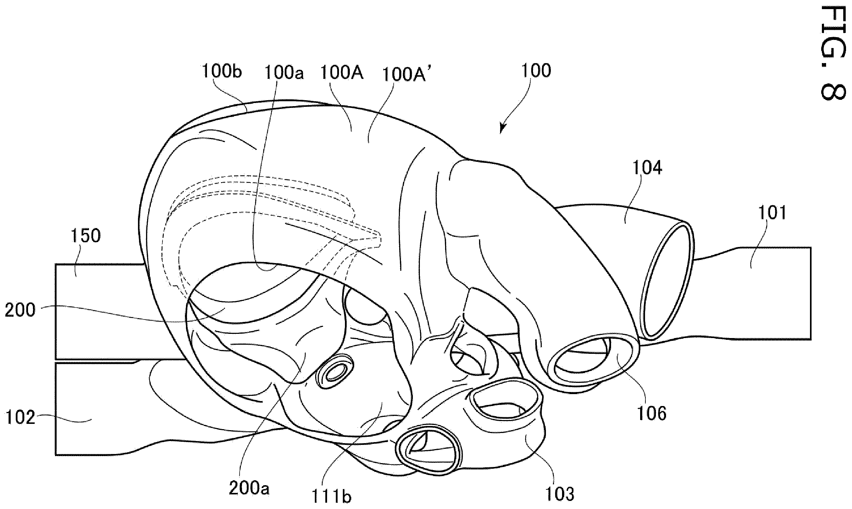

The holder 200 of the present embodiment is formed from a material having higher hardness (a hard resin or the like) than the main body 100A of the heart model formed from a material having flexibility, and is configured to be easily detachable without being deformed or the like at a predetermined site. Specifically, an opening 130a is formed in the installation region of the interventricular septum 130, and the holder 200 is formed in the same shape as the formed opening 130a. Furthermore, in the outer shell 100A' of the main body 100A, an opening part 100a for holder attachment and detachment is formed (Fig. 7 shows a state in which the holder 200 has been detached, Fig. 8 shows a state in which the holder 200 is attached, and the opening part 100a of the present embodiment is formed on the outer shell 100A' on the right ventricle 110b side), so that the holder 200 can be attached and detached with respect to the opening 130a of the interventricular septum 130. Incidentally, it is preferable that a knob 200a is formed in the holder 200 so that attachment and detachment manipulation with respect to the opening 130a can be easily carried out. By grasping the knob 200a, the holder 200 can be inserted into the left ventricle 111b through the opening part 100a, and an attachment and detachment manipulation of the holder with respect to the opening 130a can be easily carried out.

The holder 200 is formed in a plate shape so as not to protrude significantly on the right ventricle side when the holder is fitted into the opening 130a of the interventricular septum 130. The holder 200 is a separate body and holds a soft material 201 (hereinafter, a site where such a soft material is disposed on the surface will be referred to as flexible part 201). It is preferable that this material 201 is formed from a material softer than the main body 100A of the heart model. Since the locking part of a leadless pacemaker is fixed to the flexible part 201, the holder 200 is attached such that the flexible part 201 faces toward the right ventricle side. Accordingly, the material of the flexible part 201 is not particularly limited as long as it is a material having sufficient flexibility on the surface so that the locking part of a leadless pacemaker can be fixed thereto. In this case, it is preferable that the flexible part 201 is formed from a material having unevenness on the surface (unevenness means a state with a roughened surface, such as being not smooth but rough) so that a catheter can be easily locked. Furthermore, a material with which there is no feeling of discomfort even when a contrast agent is injected under X-ray fluoroscopy, and a feeling of manipulation similar to that of an actual operation can be obtained, is preferable. In addition, it is preferable that the flexible part 201 has a larger area than the opening.

The flexible part 201 of the present embodiment is constructed of a hollow material so that the locking part of a leadless pacemaker is easily caught, and a situation in which the locking part is engaged with and fixed to the heart tissue can be accurately simulated. That is, as the entirety is constructed of a hollow material, a large number of porous minute holes are present in the surface region, and these holes bring forth flexibility and surface unevenness. Specifically, as such a hollow material, for example, porous sponge can be used. The flexible part 201 formed from such a material can be fixed to depressions 200b formed on the holder 200 by means of adhesion, press fitting, or the like. Therefore, even when the surface portion of the flexible part 201 is damaged due to repeated practice, the flexible part 201 itself can be easily exchanged, and if necessary, it is also possible to use a flexible part of a different material.

It is preferable that a recess 201a corresponding to the shape of the leadless pacemaker is formed on the surface of the flexible part 201 so that when the leadless pacemaker is brought close to the surface, a sensation similar to that of an actual operation can be obtained without any slipping or displacement occurring on the surface. In this case, the recess 201a can be formed by denting the surface portion of the flexible part 201 constructed of a material such as described above, and as a result, any sliding or bouncing movement more than necessary of the leadless pacemaker can be suppressed as in the surface state of an actual heart, so that it is possible to practice the manipulation of fixing the locking part as equivalent to an actual operation.

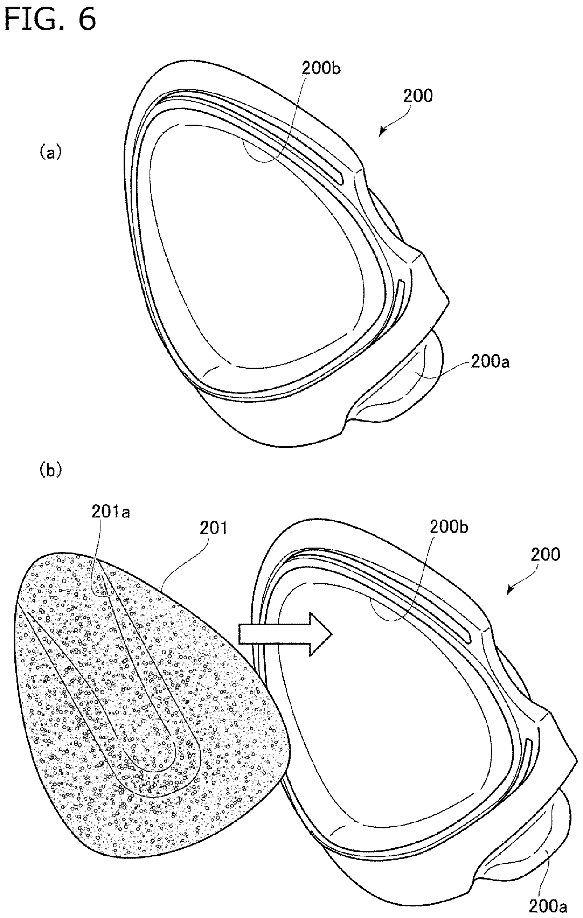

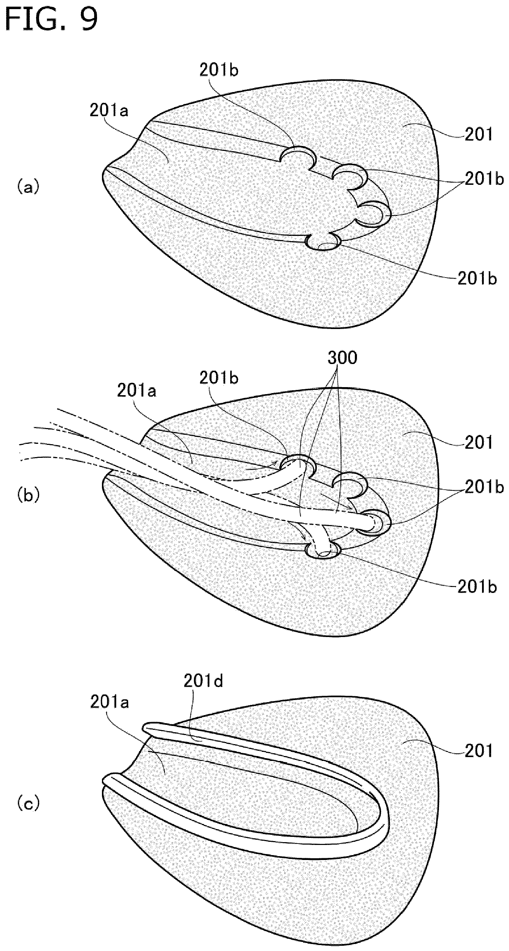

A recess 201a was formed on the surface of the flexible part 201 shown in Fig. 6 so that the leadless pacemaker can be easily locked; however, with regard to the method of forming the recess, various modifications can be made. For example, in a first Modification Example shown in Fig. 9(a), one or more (four sites in the diagram) pits 201b are formed along with the recess, around the insertion back side of the recess 201a. With regard to such pits 201b, as shown in Fig. 9(b), when the leadless pacemaker 300 is brought as conveyed by a catheter, as the tip portions of the leadless pacemaker 300 penetrate into the pits 201b, the sliding or bouncing movement of the leadless pacemaker 300 is effectively suppressed, and it is possible to practice the manipulation of fixing the locking part as equivalent to an actual operation.

In a second Modification Example shown in Fig. 9(c), an example of forming a protrusion 201d in addition to the recess 201a is shown. The protrusion 201d is formed so as to surround the above-described recess 201a, and by forming such a protrusion, drifting of the leadless pacemaker is restricted, and it is possible to make it difficult for the leadless pacemaker to be shifted from the recess 201a. Furthermore, when such a protrusion is formed, it is preferable that the protrusion 201d is formed such that the angle of elevation thereof with respect to the bottom face of the holder (surface of the flexible part) is 90 degrees or less (inclined so as to cover the recess 201a where the leadless pacemaker penetrates, from above). By forming an inclined protrusion 201d as such, when the leadless pacemaker is brought near to a predetermined site, any sliding or bouncing movement more than necessary of the leadless pacemaker can be effectively suppressed as in the surface state of an actual heart, and it is possible to practice the manipulation of fixing the locking part as equivalent to an actual operation.

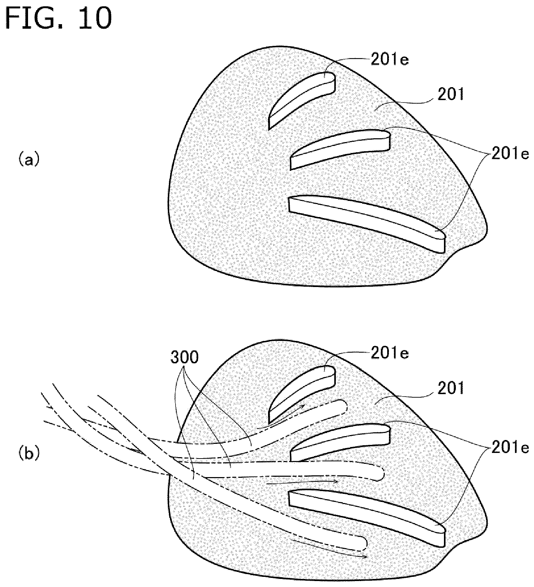

The protrusion 201d may be formed without having a recess. For example, in a third Modification Example shown in Fig. 10(a), a plurality of protrusions 201e are formed (three sites in the diagrams) on the surface of the flexible part 201 along the direction in which the leadless pacemaker is brought as inserted. The leadless pacemaker that is brought as inserted can be locked between the protrusions 201e as shown in Fig. 10(b), and when a plurality of protrusions 201e are formed as such, the leadless pacemaker to be inserted can be locked at a plurality of positions. In this case, recesses may be formed between the protrusions 201e, or recess or pits where the leadless pacemaker fits in may be appropriately formed along the protrusions. That is, it is preferable that the protrusions are disposed in the form of surrounding a recess formed such that a leadless pacemaker penetrates therein. Furthermore, it is preferable that the shape of the protrusions is a shape that surrounds the recess except for the place where the catheter enters or exits, and it is also acceptable that the flesh in the human body is reproduced, and the protrusions are disposed in a stripe pattern in accordance with the shape of the tip of the catheter, or even the shape of the tip of the leadless pacemaker. By providing such protrusions 201d (201e) and a recess 201a or pits 201b on the surface of the flexible part 201, when the leadless pacemaker is brought near to a predetermined site, any sliding or bouncing movement more than necessary of the leadless pacemaker can be suppressed as in the surface state of an actual heart, and it is possible to practice the manipulation of fixing the locking part as equivalent to an actual operation.

Incidentally, when forming the recesses, pits, and protrusions, any one or more of them may be formed on the surface of the flexible part, and the shape, the number of pieces to be formed, the pattern of disposition, and the like of the recesses, pits, and protrusions can be appropriately modified. Furthermore, the surface shape itself of the surface unevenness may be formed into a shape to which the leadless pacemaker can be easily locked.

Furthermore, holes (openings) through which a liquid can pass may be formed in the holder 200. When a large number of such openings 200h are formed, for example, on the surface where the flexible part 201 is mounted as shown in Fig. 6, the liquid can be made to flow easily inside the main body of the heart model (in the present embodiment, a fluid can flow easily between the left ventricle and the right ventricle, and in a case where a contrast agent or the like is used, retention can be prevented).

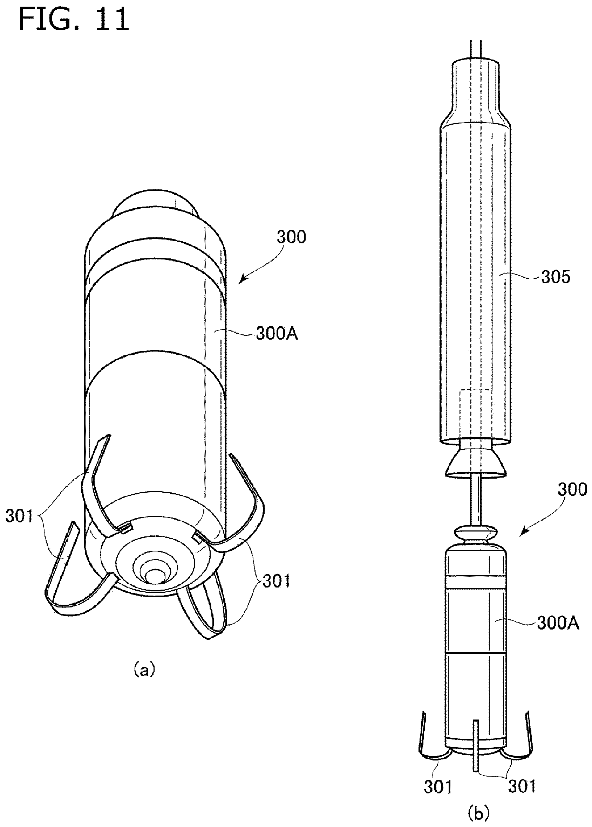

Fig. 11 is a diagram illustrating the configuration of a leadless pacemaker used in the above-described heart model 100. The leadless pacemaker 300 used in the present embodiment is referred to as MICRA (registered trademark) manufactured by Medtronic plc, and an electric circuit, an electrode, and a battery are incorporated inside a main body 300A configured to have a cylindrical shape with a total length of 25.9 mm, a diameter of 6.7 mm, and a volume of about 1 cc. Furthermore, at the tip of the main body 300A, tines 301 that serve as a locking part are formed at an interval of about 90 degrees along the circumferential direction.

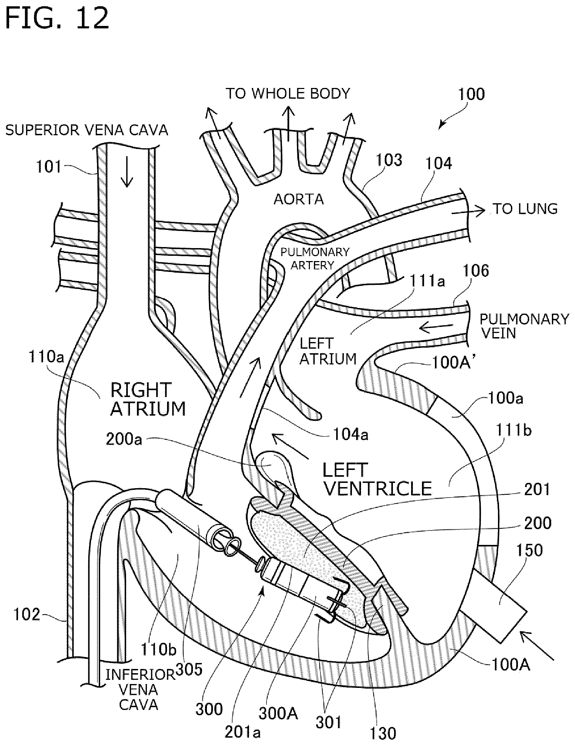

The leadless pacemaker 300 configured as such is accommodated inside a device cup 305 provided at the tip of a dedicated delivery catheter system (not shown in the diagram), inserted into the inferior vena cava through the groin, and conveyed into the right ventricle via the right atrium. Inside the right ventricle 110b, installation of the leadless pacemaker 300 can be practiced by, while placing the device cup 305 at the tip at a predetermined position of the interventricular septum (in the present embodiment, as shown in Fig. 12, the recess 201a portion of the flexible part 201 of the holder 200) by manipulating a handle at hand, pressing the main body 300A of the leadless pacemaker 300 accommodated inside the device cup 305 against the flexible part 201, and checking whether at least two of the tines 301 are locked. In this case, when the tines 301 are deformed to face outward by applying a tensile force to the main body 300A, it can be confirmed that the deformed tines are engaged with the flexible part 201, and when the tines keep a hook shape without change, it can be confirmed that the tines are not engaged with the flexible part 201.

According to the above-described heart model 100 and the catheter simulator 1, it is possible to accurately train an operation for indwelling the leadless pacemaker 300 in the right ventricle. In this case, the heart model 100 can be caused to float in a transparent container 10, training can be carried out while visually recognizing the heart model 100, the container 10 is covered with a cover, a contrast agent is allowed to flow, and training can be carried out while looking at the monitor. Furthermore, in the present embodiment, as shown in Fig. 5 and Fig. 12, when the main body 100A of the heart model 100 is provided with an inflow port 150, and a liquid communication part 104a that opens to the left ventricle 111b is formed in the pulmonary artery 104 communicating with the right ventricle 110b, a flow of a liquid as indicated by the arrow is created inside the main body, and the same flow of blood as that in an actual heart can be generated. In addition, when a contrast agent is used, the contrast agent does not stay inside the main body 100A due to the flow of liquid inside the heart. Incidentally, for a similar purpose, a liquid communication part may also be formed between the pulmonary artery 104 and the aorta 103.

As described above, when the heart model 100 is formed from a transparent or semi-transparent material, it is possible to practice while checking the movement of the leadless pacemaker; however, in some cases, it can be thought that the trainee may wish to practice in a situation that is more easily visually recognizable. Therefore, as shown in Fig. 4, it is preferable to create cutting lines 100b for forming an opening region to the outer shell 100A' of the main body 100A so that the movement of the catheter inserted inside the main body 100A can be visually recognized. The user can visually recognize the inner part of the main body by cutting out the outer shell 100A' along the cutting lines 100b as necessary, and when performing a simulation, the user can easily figure out the movement of the leadless pacemaker 300.

As described above, according to the above-mentioned catheter simulator 1 and the heart model 100, an operation related to the installation of a leadless pacemaker can be conveniently practiced. Furthermore, in the above-described example, practice was achieved in an environment in which the container 10 was filled with a liquid; however, it is also possible to practice the catheter manipulation in a dry environment without filling the container 10 with a liquid. Furthermore, depending on the configuration of the leadless pacemaker and the site of installation in the heart, it may be considered to change the route for approaching into the main body of the heart model (simulated blood vessel route to be used) or the locking method (method for manipulating a catheter); however, the site of installation of the holder 200 having a flexible part 201 to which a catheter can be locked can also be appropriately changed in accordance to the change in the route or locking method. For example, an opening for attaching or detaching the holder 200 may be formed directly on the outer shell 100A' of the main body 100A.

Furthermore, the positions of arrangement of the above-described connection part and introduction part of the container 10 can be appropriately modified, and the position of formation of an inflow port for causing a liquid to flow into the main body of the heart model to be installed in the container can also be appropriately modified. For example, with regard to the inflow of a liquid, an inflow port may be formed in portions other than the simulated blood vessel passage in the outer shell of the main body, such as the right atrium 110a or the right ventricle 110b, or it may be configured such that an opening is formed in the simulated blood vessel passage to discharge the liquid into the container.

Thus, regarding the catheter maneuvers, placement of a leadless pacemaker has been described; however, even for a temporary pacing catheter that has been conventionally used, training can be similarly carried out by using the present model.

Next, a myocardial biopsy maneuvers that can be trained by utilizing the above-described heart model 100 and catheter simulator 1 will be described.

Myocardial biopsy is an examination method of percutaneously collecting myocardial tissue by using a catheter, and in order to perform histological diagnosis for patients with cardiac hypofunction and cardiomyopathy of unknown cause, or for patients after heart transplantation, myocardial biopsy is an unavoidable examination. These days, myocardial biopsy is also applied to performing a genetic diagnosis from collected tissues and has greatly contributed to the diagnosis of patients with heart diseases and the decision of treatment policy. With regard to the examination, it has been reported that right ventricular perforation may occur with a probability of approximately 1%, and cases of death from cardiac tamponade have also been reported. Therefore, myocardial biopsy is a very useful examination while it is a risky examination, and operators who perform the maneuvers are required to have a high level of technique.

On the other hand, according to the reports of the Japanese Circulation Society, the number of cases in Japan was about 7,000 as of year 2015, which is a clearly small number of cases even as compared with 500,000 cases of CAG (coronary angiography) and 250,000 cases of PCI (coronary angioplasty). That is, this number means that the maneuver is a maneuver for which the number of cases to be experienced for each doctor alone is small, and there is a mismatch that even though it is considered to be an examination with a relatively high risk, there are few opportunities for the doctors who perform the maneuvers to accumulate experience.

In many maneuvers for myocardial biopsy, a catheter is inserted into the right ventricle through the internal jugular vein via the superior vena cava, or through the femoral vein via the inferior vena cava, and myocardial biopsy forceps are delivered into the right ventricle by way of passing through the catheter.

The maneuvers are very similar to the maneuvers for embedding a leadless pacemaker of the above-mentioned embodiment; however, the major difference is that in the maneuvers for installing a leadless pacemaker, the leadless pacemaker is implanted at the interventricular septum (portion where the holder 200 is installed), whereas in the myocardial biopsy, a myocardial biopsy is delivered through the right ventricle side to the interventricular septum, and myocardial tissue is collected from the interventricular septum.

Therefore, training can be achieved by utilizing the same heart model as that in the case of the leadless pacemaker; however, it is not necessary to fix the locking part as in the leadless pacemaker, and rather it is necessary to cut off a portion into an appropriate size.

In order to realize this, it is preferable that the flexible part 201 that is fixed to the above-described holder 200 is constructed of porous sponge or a soft resin that can be grabbed with myocardial biopsy forceps. However, it is preferable that the flexible part 201 is formed from a material softer than that of the main body of the heart model so that there is no feeling of discomfort even when a contrast agent is injected under X-ray fluoroscopy, and a feeling of manipulation similar to that of an actual operation can be obtained.

In clinical sites, maneuvers are performed under X-ray fluoroscopy, and for training, a transparent heart is preferred so that the behavior of a catheter can be checked during maneuvers, while it is more preferable that the free wall located on the body side of the interventricular septum is opened.

For these reasons, it is preferable that the heart main body, the holder 200, and the flexible part 201 attached to the holder are formed from materials having high X-ray transparency. Furthermore, the most important complication for the maneuvers of myocardial biopsy is perforation, and it is preferable that it can be checked whether the tissue could be collected from a correct position during training. Accordingly, it is preferable that the flexible part 201 is formed from a material whose difference from the heart main body can be seen at a glance, that is, a material having a different color. Since it is preferable that the heart main body is transparent as described above, it is preferable that the flexible part has a color that is easily perceivable by the trainee or the instructor, such as red color for example.

On the occasion of the myocardial biopsy maneuvers, the myocardial biopsy forceps are manipulated using a sheath with a J-shaped tip so that the tip part of the myocardial biopsy forceps are in a perpendicular direction to the interventricular septum; however, in real clinical practice, the myocardial biopsy forceps are not necessarily perpendicular to the interventricular septum and are often slightly slanted. Therefore, it is preferable that the flexible part 201 is formed from a material that is not slippery.

In clinical sites, in order to conduct training within time constraints, it is required that the simulator can be prepared rapidly, and that the time taken to move from completion of one round of maneuver to the next round of maneuver is shortened as far as possible. As described above, with the present simulator, a plurality of trainings can be efficiently carried out by exchanging the holder part, and preparation can be made in a short period of time.

## Claims

1. A heart model (100) comprising:
a main body (100A) having one or more cavities in the inner part;
a simulated blood vessel passage being contiguous to the main body (100A) and allowing insertion therethrough of a catheter,
**characterized in that**
the heart model (100) comprises a holder (200) being detachably installed inside the main body (100A) and including a flexible part (201),
wherein the flexible part (201) is formed from a porous material having surface unevenness on a surface thereof or from a soft resin mounted on the holder (200),
wherein the holder (200) including the flexible part (201) is detachably installed at a position of an interventricular septum (130) inside the main body (100A), and
wherein the one or more cavities include a right atrium (110a), and a right ventricle (110b) and a left ventricle (111b) divided by the interventricular septum (130),
wherein the simulated blood vessel passage comprises a superior vena cava (101) or an inferior vena cava (102), or both of them, communicating with the right atrium (110a),
in the interventricular septum (130), an opening (130a) detachably fitting the holder (200) is formed such that the flexible part (201) is exposed inside the right ventricle (110b), and
in an outer shell (100A') of the main body, an opening part (100a) for holder attachment and detachment is formed to allow the holder (200) to be installed in the opening (130a) of the interventricular septum (130).

2. The heart model (100) according to claim 1, wherein the flexible part (201) is mounted on the holder (200) and has an area larger than the opening (130a) of the interventricular septum (130).

3. The heart model (100) according to claim 1 or 2, wherein
in the outer shell (100A') of the main body (100A), an inflow port (150) opened to the left ventricle (111b) side and protruding to the outside is formed,
a pulmonary artery (104) communicating with the right ventricle (110b) is provided, and
in the pulmonary artery (104), a liquid communication part (104a) opened to the left ventricle (111b) or to an aorta (103), or to both of them is formed.

4. The heart model (100) according to any one of claims 1 to 3,
wherein holes (200h) allowing passage of a liquid are formed in the holder (200).

5. The heart model (100) according to any one of claims 1 to 4,
wherein in the outer shell (100A') of the main body (100A), cutting lines (100b) for forming an opening region allowing visual recognition of the movement of a catheter inserted inside the main body (100A) are created.

6. An use of the heart model (100) according to any one of claims 1 to 5, for a simulation of an operation for installing a leadless pacemaker (300) or a simulation of a myocardial biopsy maneuver.

7. The use according to claim 6,
wherein any one or more of a recess (201a), a pit (201b), and a protrusion (201d, 201e) are formed on the surface of the flexible part (201) corresponding to the shape of the leadless pacemaker (300).

8. A catheter simulator comprising:
a container (10) equipped with an accommodation part (10a) accommodating the heart model (100) according to any one of claims 1 to 5 in a state of being filled with a liquid;
a connection part (11a, 11b, 11c, 13a) provided in the container (10) and holding the heart model (100); and
an introduction part (11a', 11c', 13a') provided in the container (10) and allowing insertion therethrough of a catheter,
wherein the container (10) includes a holding part for holding the heart model (100) such that the simulated blood vessel passage is connected to the introduction part (11a', 11c', 13a').

9. The catheter simulator according to claim 8, wherein
the catheter simulator includes a pump (50) for circulating the liquid filled in the container (10), and
the pump (50) generates a pulsatile flow to an internal space of the heart model (100) held in the container (10) in a state of being filled with a liquid.

10. The catheter simulator according to claim 8, wherein
the catheter simulator includes a pump (50) for circulating the liquid filled in the container (10),
and
on a side wall (11) of the container (10), an outlet port (11d) for discharging the liquid in the accommodation part (10a) to the pump (50) is formed.

## Patentansprüche

1. Herzmodell (100), umfassend:
einen Hauptkörper (100A) mit einem oder mehreren Hohlräumen in dem inneren Teil;
einen an den Hauptkörper (100A) anschließenden simulierten Blutgefäßkanal, durch den ein Katheter einführbar ist,
**dadurch gekennzeichnet, dass**
das Herzmodell (100) eine lösbar in dem Hauptkörper (100A) angebrachte Halteeinrichtung (200) umfasst, die einen flexiblen Abschnitt (201) aufweist,
wobei der flexible Abschnitt (201) aus einem an seiner Oberfläche Oberflächenunebenheiten aufweisenden porösen Material oder einem an der Halteeinrichtung (200) angebrachten weichen Harz gebildet ist,
wobei die den flexiblen Abschnitt (201) aufweisende Halteeinrichtung (200) lösbar an einer Stelle eines Ventrikelseptums (130) in dem Hauptkörper (100A) angebracht ist und
wobei die eine oder die mehreren Hohlräume ein rechtes Atrium (110a) sowie ein rechtes Ventrikel (110b) und ein linkes Ventrikel (111b) umfassen, die durch das Ventrikelseptum geteilt sind (130),
wobei der simulierte Blutgefäßkanal eine obere Hohlvene (101) oder eine untere Hohlvene (102) oder beide umfasst, die mit dem rechten Atrium (110a) kommuniziert bzw. kommunizieren,
in dem Ventrikelseptum (130) eine die Halteeinrichtung (200) lösbar aufnehmende Öffnung (130a) derart ausgebildet ist, dass der flexible Abschnitt (201) in dem rechten Ventrikel (110b) freiliegt, und
in einem Mantel (100A') des Hauptkörpers ein Öffnungsabschnitt (100a) für die Anbringung und Entfernung der Halteeinrichtung ausgebildet ist, über welche die Halteeinrichtung (200) in der Öffnung (130a) des Ventrikelseptums (130) anbringbar ist.

2. Herzmodell (100) nach Anspruch 1, wobei der flexible Abschnitt (201) an der Halteeinrichtung (200) angebracht ist und eine größere Fläche als die Öffnung (130a) des Ventrikelseptums (130) aufweist.

3. Herzmodell (100) nach Anspruch 1 oder 2, wobei
in dem Mantel (100A') des Hauptkörpers (100A) ein zur Seite des linken Ventrikels (111b) offener und nach außen vorspringender Einlassanschluss (150) ausgebildet ist,
eine mit dem rechten Ventrikel (110b) kommunizierende Pulmonalarterie (104) vorgesehen ist und
in der Pulmonalarterie (104) ein zu dem linken Ventrikel (111b) oder zu einer Aorta (103) oder zu beiden hin offener Fluidkommunikationsabschnitt (104a) ausgebildet ist.

4. Herzmodell (100) nach einem der Ansprüche 1 bis 3,
wobei von einer Flüssigkeit durchströmbare Löcher (200h) in der Halteeinrichtung (200) ausgebildet sind.

5. Herzmodell (100) nach einem der Ansprüche 1 bis 4,
wobei in dem Mantel (100A') des Hauptkörpers (100A) Schnittlinien (100b) zur Ausbildung eines Öffnungsbereichs gebildet sind, durch welchen die Bewegung eines in den Hauptkörper (100A) eingeführten Katheters visuell erkennbar ist.

6. Verwendung des Herzmodells (100) nach einem der Ansprüche 1 bis 5 für eine Simulation einer Operation zum Einsetzen eines kabellosen Herzschrittmachers (300) oder eine Simulation eines Myokardbiopsiemanövers.

7. Verwendung nach Anspruch 6,
wobei an der Oberfläche des flexiblen Abschnitts (201) eine Ausnehmung (201a), eine Mulde (201b) und/oder ein Vorsprung (201d, 201e) ausgebildet ist bzw. sind, der bzw. die der Form des kabellosen Herzschrittmachers (300) entspricht bzw. entsprechen.

8. Kathetersimulator, umfassend:
einen Behälter (10), der mit einem Aufnahmeabschnitt (10a) versehen ist, der in einem mit einer Flüssigkeit gefüllten Zustand das Herzmodell (100) nach einem der Ansprüche 1 bis 5 aufnimmt;
einen in dem Behälter (10) vorgesehenen Verbindungsabschnitt (11a, 11b, 11c, 13a), der das Herzmodell (100) hält; und
einen in dem Behälter (10) vorgesehenen Einführabschnitt (11a', 11c', 13a'), durch den ein Katheter einführbar ist,
wobei der Behälter (10) einen Halteabschnitt aufweist, der dazu ausgebildet ist, das Herzmodell (100) derart zu halten, dass der simulierte Blutgefäßkanal mit dem Einführabschnitt (11a', 11c', 13a') verbunden ist.

9. Kathetersimulator nach Anspruch 8, wobei
der Kathetersimulator eine Pumpe (50) zum Umwälzen der in den Behälter (10) gefüllten Flüssigkeit umfasst und
die Pumpe (50) eine pulsierende Strömung zu einem Innenraum des in dem mit Flüssigkeit gefüllten Behälter (10) gehaltenen Herzmodells (100) erzeugt.

10. Kathetersimulator nach Anspruch 8, wobei
der Kathetersimulator eine Pumpe (50) zum Umwälzen der in den Behälter (10) gefüllten Flüssigkeit umfasst und
an einer Seitenwand (11) des Behälters (10) ein Auslassanschluss (11d) zur Ableitung der Flüssigkeit in dem Aufnahmeabschnitt (10a) zu der Pumpe (50) ausgebildet ist.

## Revendications

1. Modèle de cœur (100) comprenant :
un corps principal (100A) comportant une ou plusieurs cavités dans la partie interne ;
un passage de vaisseau sanguin simulé contigu au corps principal (100A) et permettant l'insertion d'un cathéter à travers celui-ci,
**caractérisé en ce que**
le modèle de cœur (100) comprend un support (200) installé de manière amovible à l'intérieur du corps principal (100A) et comprenant une partie flexible (201),
dans lequel la partie flexible (201) est formée à partir d'un matériau poreux présentant des irrégularités de surface sur une surface de celui-ci ou à partir d'une résine souple montée sur le support (200),
dans lequel le support (200) comprenant la partie flexible (201) est installé de manière amovible à une position d'un septum interventriculaire (130) à l'intérieur du corps principal (100A), et
dans lequel la ou les cavités comprennent un oreillette droite (110a) et un ventricule droit (110b) et un ventricule gauche (111b) séparés par le septum interventriculaire (130),
dans lequel le passage de vaisseau sanguin simulé comprend une veine cave supérieure (101) ou une veine cave inférieure (102), ou les deux, communiquant avec l'oreillette droite (110a),
dans le septum interventriculaire (130), une ouverture (130a) s'adaptant de manière amovible au support (200) est formée de telle sorte que la partie flexible (201) soit exposée à l'intérieur du ventricule droit (110b), et
dans une enveloppe extérieure (100A') du corps principal, une partie d'ouverture (100a) pour la fixation et le détachement du support est formée pour permettre au support (200) d'être installé dans l'ouverture (130a) du septum interventriculaire (130).

2. Modèle de cœur (100) selon la revendication 1, dans lequel la partie
flexible (201) est montée sur le support (200) et a une surface supérieure à l'ouverture (130a) du septum interventriculaire (130).

3. Modèle de cœur (100) selon la revendication 1 ou 2, dans lequel
dans l'enveloppe extérieure (100A') du corps principal (100A), un orifice d'entrée (150) ouvert vers le côté du ventricule gauche (111b) et faisant saillie vers l'extérieur est formé,
une artère pulmonaire (104) communiquant avec le ventricule droit (110b) est prévue, et
dans l'artère pulmonaire (104), une partie de communication de liquide (104a) ouverte vers le ventricule gauche (111b) ou vers une aorte (103), ou vers les deux, est formée.

4. Modèle de cœur (100) selon l'une quelconque des revendications 1 à 3,
dans lequel des trous (200h) permettant le passage d'un liquide sont formés dans le support (200).

5. Modèle de cœur (100) selon l'une quelconque des revendications 1 à 4,
dans lequel, dans l'enveloppe extérieure (100A') du corps principal (100A), des lignes de découpe (100b) sont créées pour former une région d'ouverture permettant la reconnaissance visuelle du mouvement d'un cathéter inséré à l'intérieur du corps principal (100A).

6. Utilisation du modèle de cœur (100) selon l'une quelconque des revendications 1 à 5 pour une simulation d'une opération d'installation d'un stimulateur cardiaque sans fil (300) ou une simulation d'une manœuvre de biopsie myocardique.

7. Utilisation selon la revendication 6,
dans lequel un ou plusieurs éléments parmi un évidement (201a), un creux (201b) et une saillie (201d, 201e) sont formés sur la surface de la partie flexible (201) correspondant à la forme du stimulateur cardiaque sans fil (300).

8. Simulateur de cathéter comprenant :
un récipient (10) équipé d'une partie de logement (10a) logeant le modèle de cœur (100) selon l'une quelconque des revendications 1 à 5 dans un état rempli d'un liquide ;
une partie de connexion (11a, 11b, 11c, 13a) prévue dans le récipient (10) et supportant le modèle de cœur (100) ; et
une partie d'introduction (11a', 11c', 13a') prévue dans le récipient (10) et permettant l'insertion à travers celui-ci d'un cathéter,
dans lequel le récipient (10) comprend une partie de support pour supporter le modèle de cœur (100) de telle sorte que le passage de vaisseau sanguin simulé soit relié à la partie d'introduction (11a', 11c', 13a').

9. Simulateur de cathéter selon la revendication 8, dans lequel
le simulateur de cathéter comprend une pompe (50) pour faire circuler le liquide rempli dans le récipient (10), et
la pompe (50) génère un flux pulsatile vers un espace interne du modèle de cœur (100) supporté dans le récipient (10) dans un état rempli de liquide.

10. Simulateur de cathéter selon la revendication 8, dans lequel
le simulateur de cathéter comprend une pompe (50) pour faire circuler le liquide rempli dans le récipient (10), et
sur une paroi latérale (11) du récipient (10), un orifice de sortie (11d) pour évacuer le liquide dans la partie de logement (10a) vers la pompe (50) est formé.
